# EUROPEAN PATENT APPLICATION

(11) **EP 4 106 060 A1**
(43) Date of publication of application: **21.12.2022**
(21) Application number: 21305843.1
(22) Date of filing: 18.06.2021
(51) Int. Cl.: H01M 8/18

(54) **BIOBASED AQUEOUS ORGANIC ELECTROLYTE FOR AQUEOUS ORGANIC REDOX FLOW BATTERY**

(71) Applicant: Kemiwatt, 35708 Rennes (FR); PILI, 31077 Toulouse Cedex 4 (FR)
(72) Inventor: GODET-BAR, Thibault, 35700 RENNES (FR); BOISSONNAT, Guillaume, 31077 TOULOUSE CEDEX 4 (FR)
(74) Representative: Lavoix

(57) **Abstract**

The present invention relates to a redox flow battery comprising at least one aqueous electrolyte comprising a fermented compound of formula (I) 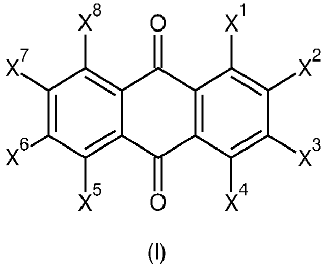 and/or an ion of compound (I),
and/or a salt of compound (I),
and/or a reduced form of the anthraquinone member of compound (I),
wherein:
X₁, X₂, X₃, X₄, X₅, X₆, X₇ and X₈ are independently selected from the group consisting of a hydrogen atom, a halogen atom, an ether group of formula -O-A, a linear, cyclic or branched, saturated or unsaturated, optionally substituted, hydrocarbon group comprising from 1 to 10 carbon atoms, a OH group, a -R₁ group and a -O-A-R₁ group,
A representing a linear, cyclic or branched, saturated or unsaturated, optionally substituted, hydrocarbon group comprising from 1 to 10 carbon atoms;
R₁ representing COOH or SO₃H;
wherein one to three of X₁, X₂, X₃, X₄, X₅, X₆, X₇ and X₈ is OH, and
wherein one and only one of X₁, X₂, X₃, X₄, X₅, X₆, X₇ and X₈ is -O-A-R₁.

The present invention relates also to a method for generating electricity with such compounds.

## Description

The present invention relates to a redox flow battery comprising one or more fermented organic molecules. In particular, the present invention relates to the use of a biobased and organic active material in an aqueous electrolyte for redox flow battery. The present invention also relates to a specific redox flow battery using such biobased and organic aqueous electrolyte, and a method for storing and producing current at competitive cost and with low carbon footprint.

### Prior art

World energy needs are growing along with greenhouse gas emissions (GHGE), accelerating the global warming. A large part of it comes from the direct consumption of fossil fuels (petroleum, coal, natural gas) into electricity and heat production (∼25%). The transition to low cost and low footprint energy production is engaged with the rising development of renewable energies which intermittency needs to be buffered by its combination with a stationary energy storage system. While renewable energy production devices (photovoltaic and wind turbines) have become competitive towards fossil energies, relevant commercial stationary batteries expected to be used in synergy with them suffer from fluctuating prices (vanadium ore in vanadium redox flow batteries "VRFB") and security defaults (explosion of lithium-ion "Li-ion" batteries) along with releasing important GHGE during their production, in particular during ore extraction (vanadium, lithium, cobalt...) and ore refinement processes.

Redox flow batteries (RFB) are flow batteries that employ a couple of redox compounds (active materials) in each half-cell electrolyte. The electrolytes (energy storage) are pumped into electric converters called stacks (power units), where they are charged or discharged. Considering a standard 15 kW/5 hours AORFB (Aqueous Organic RBF) system using standard electrolytes (dihydroxy-anthraquinone *versus* ferrocyanide) at pH 13, excluding the water and the electrical parts (the power converting system "PCS", the battery management system "BMS"...), the battery weight can be divided into i) the stack (∼5-10%); ii) the balance of plant including the tanks (∼15-20%) ; ii) the salts (∼5-15%) and ; iv) the active materials (∼60-70%). Considering: the materials nature and the weight ratio of i) the stacks (aluminium end-plates -1-3%, graphite plates -1-3%, hydrocarbonated plastic -1-3%, copper current collectors ∼0.1-1.0%, hydrocarbon-based membranes <0.2%) and ii) the balance of plant including the tanks (hydrocarbonated plastic ∼15-20%); the carbon footprint and the cost of such devices is majorly driven by those of the active materials owing to its weight ratio (∼60-70%).

To date, several alternatives have emerged concerning the nature of the redox compounds of the electrolyte, comprising the use of organic, organometallic, coordination complexes or inorganic redox compounds. i) Low-cost syntheses (i.e. AQ-2,7-DS vs ferrocyanide described by Seoul National University of Science and Technology), ii) materials with low GHGE during production (i.e. lignin from biomass used by CM Blu), iii) outstanding stabilities (i.e. DPPEAQ vs ferrocyanide described by Harvard University), iv) high power densities (i.e. AQ-2,7-DS vs bromine used by GES) and v) high energy densities (i.e. methyl viologen *vs* TEMPTMA used by Jena Batteries) are disclosed but all five criteria are never matched at the same time.

### Aims of the invention

The present invention aims to solve the technical problem of providing an aqueous electrolyte for redox flow battery displaying a low production cost allowing the whole system to reach a competitive CAPEX.

The present invention aims to solve the technical problem of providing an aqueous electrolyte for redox flow battery displaying low GHGE (GreenHouse Gas Emissions) during its production allowing bringing down the carbon footprint of the whole system.

The present invention aims to solve the technical problem of providing an aqueous electrolyte for redox flow battery displaying a high stored capacity.

The present invention aims to solve the technical problem of providing an aqueous electrolyte for redox flow battery displaying a high energy density.

The present invention aims to solve the technical problem of providing an aqueous electrolyte for redox flow battery displaying a high power density.

The present invention aims to solve the technical problem of providing an aqueous electrolyte for redox flow battery displaying a high stability and cyclability, i.e. a lower capacity loss over time.

The present invention aims to solve the technical problem of providing an aqueous electrolyte for redox flow battery displaying high performances, in particular when tested under conditions close to real systems.

The complexity of these technical problems is in particular related to the capability of solving all of them together, which the present invention proposes to solve. The present invention aims to solve the technical problem of providing an AORFB system able to reach the performances level, the stability level and the production cost necessary to make it competitive towards alternative stationary energy storage systems while decreasing its production's greenhouse gas emissions. The factor of competitiveness of the whole battery system is estimated by calculating its levelized cost of storage (LCOS). The LCOS takes into consideration the system's CAPEX, the OPEX, the total operational lifetime, the electricity costs, the discount rate. It is then expressed in terms of €/kWh/cycle and should reach <0.05 €/kWh/cycle according to the European Commission expertise to allow market penetration.

Accordingly, preferably, the present invention aims to solve the technical problem of providing a competitive AORFB system in view of these criteria.

### Detailed description of the invention

The present invention relates to an AORBF implanting a fermented anthraquinoe.

Advantageously, such fermented anthraquinone is synthesized via a one-step fermentation synthesis process and the present invention desmonstrated the technical performance of such fermented compound as an active material in the negolyte of an aqueous organic redox flow battery (AORFB). Surprisingly, AORBF of the present invention has shown special and surprising technical features allowing to solve one or more, preferably all, the above described technical problems.

The present invention relates to a redox flow battery comprising at least one aqueous electrolyte comprising a fermented compound of formula (I) 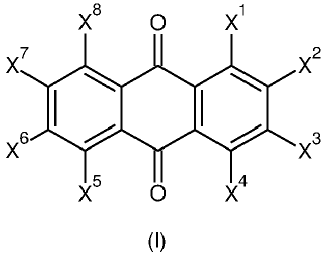 and/or an ion of compound (I),
and/or a salt of compound (I),
and/or a reduced form of the anthraquinone member of compound (I),
wherein:
X₁, X₂, X₃, X₄, X₅, X₆, X₇ and X₈ are independently selected from the group consisting of a hydrogen atom, a halogen atom, an ether group of formula -O-A, a linear, cyclic or branched, saturated or unsaturated, optionally substituted, hydrocarbon group comprising from 1 to 10 carbon atoms, a OH group, a -R₁ group and a -O-A-R₁ group,
A representing a linear, cyclic or branched, saturated or unsaturated, optionally substituted, hydrocarbon group comprising from 1 to 10 carbon atoms;
R₁ representing COOH or SO₃H;
wherein one to three of X₁, X₂, X₃, X₄, X₅, X₆, X₇ and X₈ is OH, and
wherein one and only one of X₁, X₂, X₃, X₄, X₅, X₆, X₇ and X₈ is -O-A-R₁.

In one embodiment, one and only one of X₁, X₂, X₃, X₄, X₅, X₆, X₇ and X₈ is OH and one and only one of X₁, X₂, X₃, X₄, X₅, X₆, X₇ and X₈ is -O-A-R₁.

In one embodiment, one and only one of X₃ and X₈ is OH, and one and only one of X₃ and X₈ is -O-A-R₁.

In one embodiment, X₃ is OH and X₈ is -O-A-R₁.

In one embodiment, X₃ is -O-A-R₁ and X₈ is OH.

In one embodiment, two of X₃, X₆, and X₈ are OH, and one and only one of X₃, X₆ and X₈ is -O-A-R₁.

In one embodiment, three of X₃, X₅, X₆, and X₈ are OH, and one and only one of X₃, X₅, X₆ and X₈ is -O-A-R₁.

In one embodiment, X₁ represents a linear, cyclic or branched, saturated or unsaturated, optionally substituted, hydrocarbon group comprising from 1 to 10 carbon atoms, preferably from 1 to 3 carbon atoms, and preferably represents a methyl group.

In one embodiment, A represents (CH₂)ₙ, optionally substituted, wherein n is an integer selected from 1 to 10, preferably selected from 2 to 5 and more preferably selected from 3 to 4.

In one embodiment, said -O-A-R₁ group represents O-(CH₂)ₙ-COOH or O-(CH₂)ₙ-SO₃H wherein n is from 1 to 10.

Advantageously, R₁ is selected from the group consisting of CO₂H, CO₂⁻M⁺, SO₃H and SO₃⁻M⁺, M⁺ being selected from the group consisting of Li⁺, Na⁺, K⁺ and NH₄⁺, preferably M⁺ is Na⁺ or K⁺.

Advantageously, X₂ represents a -R₁ group, and preferably represents COOH or a salt thereof.

Advantageously, said compound of formula (I) has the following structure: 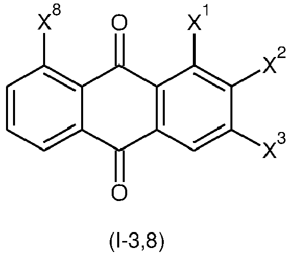 wherein X₁, X₂, X₃, and X₈ are as defined in the present invention.

Advantageously, said compound of formula (I) has the following structure: 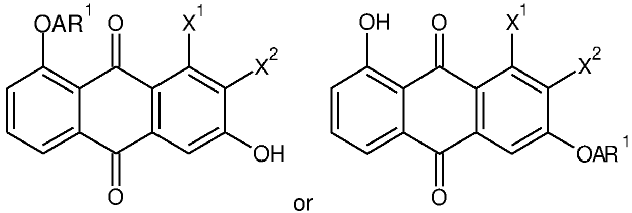 wherein X₁, X₂ and O-A-R₁ are as defined in the present invention.

Advantageously, said compound of formula (I) has the following structure: 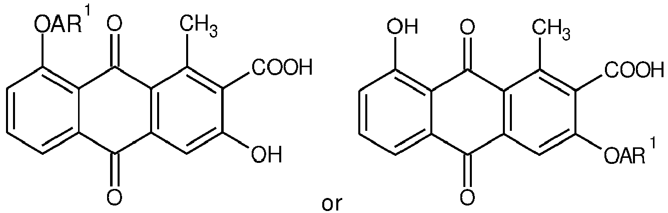 wherein O-A-R₁ are as defined in the present invention.

Advantageously, said compound of formula (I) has the following structure: 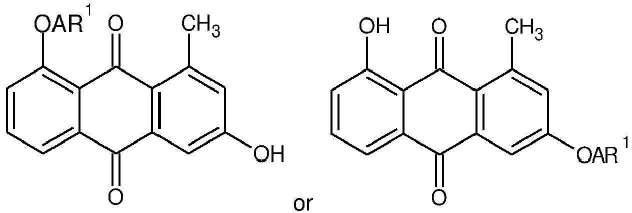 wherein O-A-R₁ is as defined in the present invention.

The expression "substituted hydrocarbon group" refers to a hydrocarbon group having one or more hydrogen atoms replaced by another/other atom(s) or group(s) of atoms and to a hydrocarbon chain comprising one or more heteroatoms in the carbon chain.

According to an embodiment, a substituted hydrocarbon group comprising from 1 to 10 carbon atoms comprises one to five heteroatoms independently selected from the group consisting of O, N, and S.

According to an embodiment, A represents a linear, cyclic or branched, saturated or unsaturated, optionally substituted, hydrocarbon group comprising from 1 to 10 carbon atoms, or represents a linear, cyclic or branched, saturated or unsaturated hydrocarbon group comprising from 1 to 10 carbon atoms and further including from 1 to 3 oxygen atoms, preferably one oxygen atom, and is for example -CH₂-CH₂-O-CH₂-CH₂-.

The compounds of the present invention are mono-substituted dihydroxy-anthraquinone refers to a dihydroxy-anthraquinone in which only one of the two hydroxyl groups is substituted by a radical different from a OH group. The mono-substituted dihydroxy- are defined by opposition to di-substituted dihydroxy-anthraquinone which comprise two substituents on the hydroxyl groups of the dihydroxy-anthraquinone. According to the invention, a di-substituted dihydroxy-anthraquinone refers to a dihydroxy-anthraquinone in which both of the two hydroxyl groups are substituted by radicals different from a OH group.

According to an embodiment, a substituted (CH₂)ₙ group wherein n is an integer selected from 1 to 10, preferably selected from 2 to 5 and more preferably selected from 3 to 4, comprises one to five heteroatoms independently selected from the group consisting of O, N, and S.

According to an embodiment, a substituted (CH₂)ₙ group wherein n is an integer selected from 1 to 10, preferably selected from 2 to 5 and more preferably selected from 3 to 4, includes from 1 to 3 oxygen atoms, preferably one oxygen atom, and is for example - CH₂-CH₂-O-CH₂-CH₂-.

Advantageously, in the compound of formula (I), n is equal to 3 or 4.

According to an embodiment, in the compound of formula (I) according to the invention, A-R₁ is selected from the group consisting of -CH₂-CH₂-O-CH₂-CH₂- COOH, - CH₂-CH₂-O-CH₂-CH₂-SO₃H, CH₂-CH₂-O-CH₂-CH₂-SO₃⁻Na⁺, -(CH₂)₃-COOH, - (CH₂)₄-COOH, -(CH₂)₃-SO₃H, -(CH₂)₄-SO₃H, -(CH₂)₃-SO₃⁻Na⁺ and -(CH₂)₄-SO₃⁻Na⁺.

In one embodiment, A-R₁ is selected from the group consisting of -(CH₂)₃-COOH, - (CH₂)₄-COOH, -(CH₂)₃-SO₃H, -(CH₂)₄-SO₃H, -(CH₂)₃-SO₃⁻Na⁺ and -(CH₂)₄-SO₃⁻Na⁺.

In one embodiment, said electrolyte comprises at least one base.

In one embodiment, the base comprises a hydroxide ion, and is preferably selected from the group consisting of potassium hydroxide, sodium hydroxide, lithium hydroxide, ammonium hydroxide, magnesium hydroxide, calcium hydroxide and any combination thereof.

In a preferred embodiment, the aqueous electrolyte of the invention comprises potassium hydroxide, sodium hydroxide or a combination of potassium hydroxide and sodium hydroxide.

In one embodiment, the base is at a concentration of 0.1 to 2 mol/L, preferably of 0.3 to 1.5 mol/L, more preferably of 0.5 to 1.1 mol/L.

According to the embodiment in which the aqueous electrolyte comprises more than one base, the concentration of the base represents the sum of the concentration of each base.

In one embodiment, said electrolyte presents a pH superior or equal to 7 and inferior or equal to 13.5, preferably superior or equal to 9 and inferior or equal to 13.5, more preferably superior or equal to 12 and inferior or equal to 13.5.

The present invention also concerns the use of an aqueous electrolyte according to the invention, as a negative electrolyte in a redox flow battery.

The present invention also concerns a redox flow battery comprising:
- a negative pole comprising an aqueous electrolyte according to the present invention, and
- a positive pole comprising a positive aqueous electrolyte.

Preferably, the positive aqueous electrolyte comprises a redox compound preferably selected from the group consisting of potassium ferrocyanide, sodium ferrocyanide, lithium ferrocyanide, ammonium ferrocyanide, magnesium ferrocyanide, calcium ferrocyanide and any combination thereof, more preferably selected from the group consisting of potassium ferrocyanide and sodium ferrocyanide and any combination thereof.

Preferably, the positive aqueous electrolyte also comprises one base. The base of the positive aqueous electrolyte preferably comprises a hydroxide ion, and is preferably selected from the group consisting of potassium hydroxide, sodium hydroxide, lithium hydroxide, ammonium hydroxide, magnesium hydroxide, calcium hydroxide and any combination thereof.

Preferably, the positive aqueous electrolyte presents a pH superior or equal to 7 and inferior or equal to 13.5, preferably superior or equal to 9 and inferior or equal to 13.5, more preferably superior or equal to 12 and inferior or equal to 13.5.

Preferably, the base comprised in the positive aqueous electrolyte is at a concentration of 0.1 to 1.5 mol/L, preferably of 0.1 to 1.0 mol/L, more preferably of 0.1 to 0.3 mol/L.

The redox flow battery also comprises a membrane. The membrane of the redox flow battery is an ion-exchange material, the membrane being preferably selected from the group consisting of a fluorinated (co)polymer, an ether ketone (co)polymer, an ether sulfone (co)polymer, an ether ether ketone (co)polymer, a sulfonated ether ether ketone (co)polymer, a benzimidazole (co)polymer and an arylene-based (co)polymer, more preferably selected from the group consisting of a sulfonated tetrafluoroethylene based fluoropolymer copolymer, a perfluorosulfonic acid ionomer, an ether ether ketone (co)polymer, a sulfonated ether ether ketone (co)polymer and a benzimidazole (co)polymer.

Preferably, the membrane of the redox flow battery has a thickness comprised between 10 µm and 90 µm, more preferably comprised between 10 and 50 µm.

The membrane is sandwiched between an anode and a cathode. Preferably, the anode and the cathode comprise porous graphite.

The present invention also concerns a method for generating electricity by at least one redox flow battery, wherein the redox flow battery is defined according to the invention.

Preferably, in the method of the invention, the aqueous electrolyte and the positive aqueous electrolyte are pumped at a flow rate comprised between 0.1 and 1.3 mL/min/cm², preferably between 0.4 and 0.8 mL/min/cm², more preferably between 0.5 and 0.6 mL/min/cm².

Preferably, in the method of the invention, the redox flow battery is discharging in a constant power mode.

Preferably, the present invention implements compounds issued from fossil-based material.

Preferably, the present invention implements biobased redox compounds i.e. extracted for example from roots of the madder plant.

Preferably, the present invention implements bio-inspired, bio-mimetic redox compounds.

Preferably, the present invention implements derivatives of biobased compounds (like lignin which is issued from black liquor, a residual material from paper industry) using standard organic chemistry and holding redox storage qualities compatible with the battery application i.e. alizarin red s which is a sulfonation of alizarin (which can be collected from a root).

As further supported by the european patent application n° 19306735.2 anthraquinone derivatives provide good power density and energy density, stability and cyclability. They suffer the inconvenient of needing several steps of chemical synthesis for their preparation. The fermented compounds of the present invention are specific over the anthraquinone derivatives of the prior art because they unexpectedly meet both the needs in terms of power density and energy density, stability and cyclability but also the CAPEX and low GHGE required for a very competitive AORBF. Overall, advantageously fermented compounds of the present invention provide an acceptable LCOS.

In the present invention, a "fermented compound" means a compound obtained by one or more fermentation steps and possibly one or more steps of modification through chemical synthesis (as opposed to fermentation).

Advantageously, fermented compounds of the present invention are prepared through a fermentation process thereby producing the active material in one step to store energy in RFBs. The compounds prepared directly from fermentation can be further modified through one or more chemical modifications (substitution, esterification and/or saponification) to provide the fermented compounds of the present invention.

Advantageously, the present invention allows a drastic diminution of synthetic steps to achieve the synthesis of an active material via fermentation process.

Advantageously, fermented compounds of the present invention are prepared through structural modification of fermented molecules using green chemistry process and are providing high performances in AORBF.

Advantageously, the present invention allows reducing drastically the production's carbon footprint of an AORFB. Advantageously, the present invention allows a drastic reduction of the active material production's carbon footprint via fermentation process in such an extent that the whole system gets its production's environmental impact substantially lower.

Advantageously, the present invention allows exploiting an organic active material synthesized via a green method (low e-factor and low carbon dioxide emissions).

Advantageously, fermented compounds of the present invention allows reducing drastically the CAPEX of an AORFB. Advantageously, the present invention provides a drastic decrease of an active material production costs via fermentation process such as, in combination with other specific AORFB features, the whole battery system becomes competitive to such an extent that it can reach the system cost (€/kWh) expected by the US DoE and by the European Commission.

Biobased building blocks synthesis.

Advantageously, the present invention involves a microbial fermentation process (fermentation), producing low carbon dioxide content, to produce anthraquinone building blocks exhibiting strong electrochemical features and excellent water solubility. Precursors of compounds according to the present invention can be obtained with this fermentation method. The precursor typically comprises two hydroxyl groups. The compounds of the present invention comprise one hydroxyl and one O-A-R₁ group. Typically, the fermented compounds of the invention are derived from the corresponding fermented precursor compounds wherein one of the two hydroxyl groups of the fermented precursor is further modified into the O-A-R₁ group.

Advantageously, a fermentation process is implemented. Typically, a fermentation process demands less than 2 steps one-pot to produce these compounds.

Classical organic chemistry process could also be used to obtain these molecules but it would take more than 5 steps (typically 7 or 8 steps) to synthesize the same molecule. Such complex synthesis path is necessarily too expensive compared to the storage's cost target that is expected at ≤100 €/kWh excluding tank cost, formulation cost, stack cost and BOP (balance-of-plant) cost.

Typical synthesis of anthraquinone would require the extraction and refining of anthracene from the anthracenic fraction of coal tar. Two distillations at 350 °C followed by subsequent recristallization would be necessary to obtain pure anthracenic fraction. Anthracene is then oxidized to anthraquinone in gaz phase using vanadium catalysts and oxygen in temperatures of 325-390 °C to yield pure anthraquinone. Another synthesis method of anthraquinone production requires the extraction and purification of naphthalene from petroleum or coal-tar naphtha fraction at 220 °C and further refining to eliminate contaminants followed by a Friedel-Crafts reaction between phthalic anhydride and benzene in the presence of 2 molar equivalents of aluminum chloride. Each ton of anthraquinone requires the use of 100 tons of petroleum, 10 tons of toxic chemicals and 1000 liters of water.

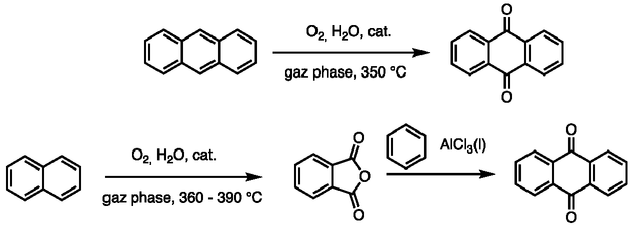

On top of anthraquinone synthesis, functionalization is required to reach compound A and compound B. 1- and 6-Hydroxylation and 3-methylation are necessary to reach compound B while an additional carbonation is necessary to reach compound A.

Typical hydroxylation of anthraquinones can occur through either sulfonation reaction followed by alkali displacement of the resulting sulfonate. Sulfonation occurs in a misture of sulfur oxide and sulfuric acid at 100-150 °C, while alkali displacement occurs in melted soda at 250 °C. Typical procedures yields a mixture of isomers that must be separated by crystallization processes. The replacement of benzene by phenol can also give rise to pure 1-hydroxyanthraquinone through the Friedel-Crafts process but the rest of the functionalization must follow.

Typical methylation involves an intermediate cyanation followed by reduction of the resulting cyano compound. Cyanation can occur either directly or by displacement of the corresponding chlorine but the preferred method is through the replacement of benzene by toluene derivatives giving rise predomionantly to the 2-methyl anthraquinone.

The synthesis of compounds A and B has been little investigated by the petrochemical industry and the compex stereochemistry requires the use of intermediate protecting or activating groups. To date, the only described synthesis relies on a Diels-Alder approach between a hydroxynaphthoquinone (prepared in two steps from dihydroxynaphthalene) and a Danishevsky's diene (prepared in three to four steps from acetoacetic acid). The synthesis has been described by Brassard et *a*/with a global yield of 3 to 5%.

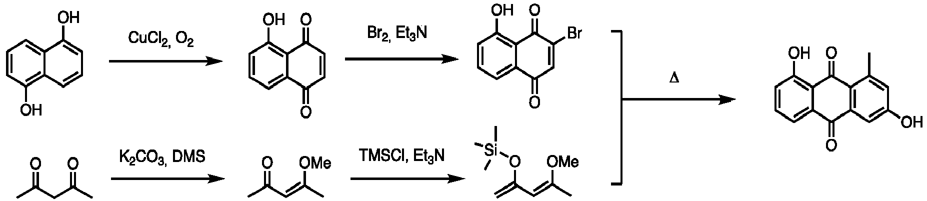

Such a multi-step synthesis is necessarily wasteful and uses complex and corrosive reagents.

In the other hand, the use of a properly designed or chosen microorganism able to produce compound A or B can considerably ease access to the molecules. Typical process would involve the use of a suitable microorganism capable to produce naturally or *via* heterologous genetic material the compound of interest in a fermentation during 2 to 7 days at 28 or 37 °C in water supplemented with sugar, nitrogen source as well as metallic salts and oligoelements in trace amounts. Most of the energetic consumption would then come from the stirring of the vessel and sugar carbon footprint, which is evaluated to be at least 10 times lower than that of petrochemical synthesis and require the use of no toxic materials nor generate toxic wastes.

Advantageously, fermented precursor compounds exhibit very interesting features in aqueous organic flow battery application as redox active material in the negolyte, though these performances do not meet simultaneously all AORFB KPI (Key Performance indicators). Advantageously, fermented compounds of the present invention allow meeting all criteria simultaneously.

### Tailoring performing redox-active materials.

Advantageously, the present invention upgrades the redox storage ability by modifying the structural structure of the fermented precursor compounds using an organic chemistry process and thereby providing a redox-active compound of the formula (I), their different embodiments and preferred structure thereof. These compounds are redox-active materials.

Advantageously, the redox-active materials of the present invention that are obtained via a low-carbon synthesis production using a microbial fermentation and an organic chemistry step exhibit simultaneously all KPls of the optimized AORFB system.

These smart materials represent a serious biobased alternative to the petrochemical industry and the inorganic chemistry (using heavy/rare/precious metals as main molecular base) industry as active materials in redox flow batteries.

Expressions such as "in one embodiment," "in another embodiment," or the like are used herein, these phrases are meant to generally reference embodiment possibilities, and are not intended to limit the invention to particular embodiment configurations. As used herein, these terms may reference the same or different embodiments that are combinable into other embodiments. Any embodiment, preferred or advantageous feature, referenced herein is freely combinable (unless otherwise stated or technically impossible) with any one or more of the other embodiments, preferred or advantageous feature, referenced herein, and any number of features of different embodiments are combinable with one another, unless indicated otherwise or technically impossible.

This detailed description is intended to be illustrative only, and should not be taken as limiting the scope of the invention.

### Figures

In the figures:
Figure 1 is a graph representing (squares) the charge capacity, (circles) the discharge capacity, (diamonds) the coulombic and (triangles) the energy efficiencies of a cell test involving a negolyte with compoundaccording to the present invention
Figure 2 represents the evolution of (dark triangles) coulombic and (dark squares) energy efficiencies according to the nominal power density during the same cycling test involving a negolyte containing compound.
Figure 3: Cyclic voltammetry (3^{rd} cycle) of the negolyte (black line) before the cycling test and (grey line) after cycling test, recorded at a scan rate of 100 mV.s⁻¹
Figure 4: ¹H NMR spectra of the negolyte, recorded in D₂O between 0 and 10 ppm, (black line) before and (grey line) after the cycling test.
Figure 5 is a graph representing (squares) the capacity retention, (diamonds) coulombic and (triangles) energy efficiencies of a cell test involving a negolyte with a compound according to the present invention. 500 cycles are presented.
Figure 6 is a graph representing the capacity available during charge and during discharge (dark, dark grey and light grey) of a cell test involving a negolyte with a compound according to the present invention.
Figure 7: Cyclic voltammetry (3^{rd} cycle) of the negolyte (black line) before the cycling test and (grey line) after cycling test, recorded at a scan rate of 100 mV.s⁻¹
Figure 8: ¹H NMR spectra of the negolyte, recorded in D₂O between 0 and 10ppm, before (above spectra) and after (bottom spectra) the cycling test.
Figure 9 is a graph representing (squares) the capacity retention, (diamonds) coulombic and (triangles) energy efficiencies of a cell test involving a negolyte with with a compound according to the present invention. 100 cycles are presented.
Figure 10: Cyclic voltammetry (3^{rd} cycle) of the negolyte (black line) before the cycling test and (grey line) after cycling test, recorded at a scan rate of 100 mV.s⁻¹
Figure 11: ¹H NMR spectra of the negolyte, recorded in D₂O between 0 and 10 ppm, (above spectra) before and (bottom spectra) after the cycling test.

### Examples

### 1. Production of Anthraquinone molecule A and B by fermentation:

Molecule A and B can be produced by fermentation of microorganisms expressing the necessary genes to their synthesis such as an octaketide synthase, a ketoreductase, an aromatase and a cyclase and fermented in a minimal medium supplemented with glucose and inoculated as known by the skilled man of the art. Alternatively, bioproduction of the molecules can be performed enzymatically *in vitro* with supplementation of precursors such as malonyl or acetyl-CoA as known by the man skilled in the art.

Production and extraction of those molecules can be performed in a recombinant E. *coli* as exhibited in patent demand WO2020161354A1 or in a modified *Streptomyces CH999* as exhibited in patent demand WO199508548, or in Taek Son Lee et al., Biorg. Med. Chem., 2007, 15, 5207 or *in vitro* as described in Carreras et al., J. Am. Chem. Soc. 1996, 118, 5158-5159.

### NMR procedure

DMSO-d6 was purchased from Euriso-top (France). 2-thiohydantoin purchased from Alfa Aesar is used internal standard to assess the sample purity as well as maleic acid purchased from Acros Organics. Product and 2-thiohydantoin were dissolved in 0.5mL of DMSO-d6. ¹H NMR spectra were recorded on BRUKER AC 300 P (300 MHz) spectrometer. Chemical shifts are expressed in parts per million. Data are given in the following order: value, multiplicity (s, singlet; d, doublet; t, triplet; q, quadruplet; p, quintuplet; m, multiplet), coupling constants J (given in Hertz), number of protons.

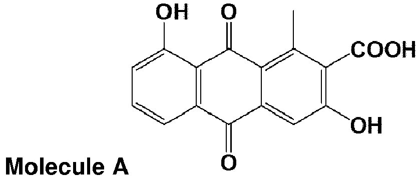

### Molecule A: NMR signature and purity

¹H NMR (300 MHz, DMSO) δ 12.87 (s, 1H), 7.72 (t, J = 7.9 Hz, 1H), 7.63 (dd, J = 7.6, 1.3 Hz, 1H), 7.59 (s, 1H), 7.33 (dd, J = 8.3, 1.3 Hz, 1H), 2.69 (s, 3H).

The purity of molecule A is estimated by quantitative ¹H NMR by using an internal standard, the 2-Thiohydantoin: ¹H NMR (300 MHz, DMSO) δ 11.64 (s, 1H), 9.83 (s, 1H), 4.04 (s, 2H). The purity is estimated at 78%.

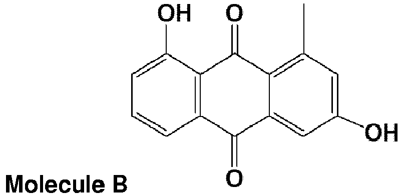

### Molecule B: NMR signature and purity

¹H NMR (300 MHz, DMSO) δ 12.87 (s, 1H), 10.99 (s, 1H), 7.61 (t, J = 7.8 Hz, 1H), 7.50 (d, J = 7.4 Hz, 1H), 7.32 (s, 1H), 7.21 (d, J = 8.2 Hz, 1H), 6.90 (s, 1H), 2.59 (s, 3H).

The purity of molecule A is estimated by quantitative ¹H NMR by using an internal standard, the 2-Thiohydantoin: ¹H NMR (300 MHz, DMSO) δ 11.64 (s, 1H), 9.83 (s, 1H), 4.04 (s, 2H). The purity is estimated at 96%.

### 2. Synthesis of molecular derivatives using an organic chemistry process. Purity measurement.

Potassium carbonate and potassium hydroxide (purity 85%) were purchased from VWR. Dry dimethylformamide and methyl 4-bromobutyrate were purchased from Acros Organics. Glacial acetic acid was purchased from Carlo Erba. Isopropanol and concentrated hydrochloric acid (37%) were purchased from Sigma Aldrich.

### Synthesis of methyl 4-((5-hydroxy-4-methyl-9,10-dioxo-9,10-dihydroanthracen-2-yl)oxy)butanoate

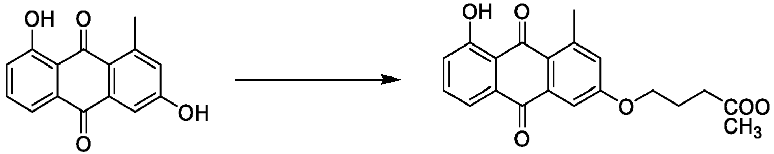

3,8-dihydroxy-1-methylanthracene-9,10-dione (5 g; 19.67 mmol; 1 eq) were dissolved in 150 mL of dry dimethylformamide with potassium carbonate (2.99 g; 21.63 mmol; 1.1 eq). The mixture is stirred at 50 °C and a solution of methyl 4-bromobutyrate (3.56 g; 19.67 mmol; 1 eq) in 50 mL of dry dimethylformamide is added dropwise over a period of 30 minutes. After stirring 44 hours at 50 °C, the mixture is poured on 125 mL of a solution of hydrochloric acid at 4%. The precipitate is isolated by filtration on a frit glass and washed with 100 mL of ultrapure water. The product is dried under vacuum for 6 hours at 40 °C, leading to the desired compound an ocher powder with a yield of 83% (purity was measured by ¹H NMR at 100% by using maleic acid as an internal standard).

¹H NMR (300 MHz, DMSO) δ 12.82 (s, 1H), 7.70 (t, J = 7.9 Hz, 1H), 7.59 (d, J = 7.4 Hz, 1H), 7.42 (d, J = 2.7 Hz, 1H), 7.30 (d, J = 8.3 Hz, 1H), 7.22 - 7.10 (m, 1H), 4.15 (t, J = 6.4 Hz, 2H), 2.68 (s, 3H), 2.02 (p, J = 6.8 Hz, 2H).

Internal standard (maleic acid): ¹H NMR (300 MHz, DMSO) δ 6.27 (s, 2H).

### Synthesis of 4-((5-hydroxy-4-methyl-9,10-dioxo-9,10-dihydroanthracen-2-yl)oxy)butanoic acid (compound B-M3CH)

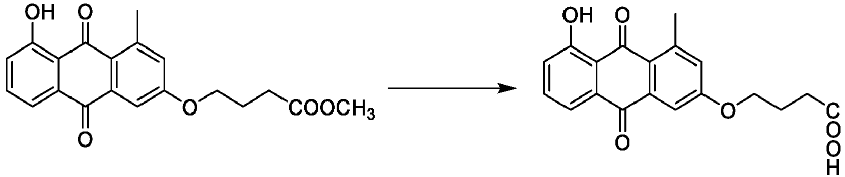

Methyl 4-((5-hydroxy-4-methyl-9,10-dioxo-9,10-dihydroanthracen-2-yl)oxy)butanoate, (5.8 g ; 16.37 mmol ; 1 eq) is suspended in 250 mL of ultrapure water and 130 mL of isopropanol. Potassium hydroxide is added (1.89 g; 28.64 mmol; 1.75 eq) and the dark red solution is stirred at 60 °C for 24 hours. The solution is poured on 700 mL of ice and 17 mL of acetic acid is added to induce precipitation of the product which is collected by filtration on a glass frit. The product is washed with 50 mL of ultrapure water and dried under vacuum for 14 hours at 40 °C. The desired product is isolated as an ocher powder with a yield of 91% (purity was measured by ¹H NMR at 95% by using maleic acid as an internal standard).

¹H NMR (300 MHz, DMSO) δ 12.82 (s, 1H), 7.71 (t, J = 7.9 Hz, 1H), 7.61 (dd, J = 7.6, 1.3 Hz, 1H), 7.45 (d, J = 2.7 Hz, 1H), 7.31 (dd, J = 8.2, 1.2 Hz, 1H), 7.21 (d, J = 2.7 Hz, 1H), 4.17 (t, J = 6.4 Hz, 2H), 2.70 (s, 3H), 2.43 (t, J = 7.3 Hz, 2H), 2.00 (p, J = 6.9 Hz, 2H).

Internal standard (maleic acid): ¹H NMR (300 MHz, DMSO) δ 6.27 (s, 2H).

### 3. Water solubilities

### Experimental procedure

The solubility of the compounds was evaluated in aqueous solution at various pH by shake-flask method. An aqueous solution (containing either KOH 1 M, NaOH 1 M, or a mixture of KOH 0.5 M and NaOH 0.5 M) is added over 100 mg of compound by 20 µL portions until complete solubilization. We obtain an approximate range of solubility values calculated as following:
Smax ∼ (m/M)/V; m = mass of evaluated compound (g); M = molar mass of evaluated compound (g.mol⁻¹); V = added volume (L).

### Results

**Table 1: solubility estimated of compounds A, B and of one compound of formula (I) (compound B-M3CH) using shake-flask method**

| Compound (I) | KOH (1 M) | NaOH (1 M) | KOH 0.5 M + NaOH 0.5 M |
|---|---|---|---|
| A | 0.48 | 0.32 | 0.49 |
| B | 0.58 | 0.5 | 0.63 |
| B-M3CH | 0.15 | 0.15 | 0.43 |

Compounds A and B are readily soluble (see table 1) in aqueous alkaline environment (>0.3 M) and present, surprisingly, an even higher solubility when mixing hydroxide salts holding potassium and sodium cations.

Compound B-M3CH of formula (I) exhibits a rather low solubility, compared to compounds A and B, in ionic environment containing one type of alkaline cation (-0.15 M). When in presence with a mix of cations (sodium and potassium cations in this example), the water solubility of this compound is increased by a factor close to 3 (∼0.43 M). This result highlights the major influence of the cationic environment on the water solubility of such compound.

### 4. Electrochemical properties at diluted state

### Experimental procedure

Cyclic voltammetry experiments were performed at a 100 mV/s scan rate for compounds A, B and B-M3CH in a solution at pH 14 (KOH 1 M).

The electrochemical experiments were conducted on an electrochemical working station (BioLogic Science Instruments VSP) at 25 °C using a three-electrodes electrochemical cell, where a Pt wire was employed at the counter electrode (CE), an Ag/AgCI/KCI saturated electrode immerged in a 1 M KOH solution protected from the cell by a porous frit glass served as the reference electrode (RE) and a rotating disc electrode (RDE) mounted with a glassy carbon (GC, 7.069 mm²) disk was used as the working electrode (WE), respectively. The potential was reported relative to the normal hydrogen electrode (NHE), which was converted from the Ag/AgCl reference electrode (0.230 V *versus* NHE). The ohmic drop between the WE and RE is determined by current interrupt experiments and leads to a value close to 0.7069 Ω.cm². All cyclic voltammetry experiments were conducted by alleviating most of this resistance (85%) using the potentiostat manual ohmic drop compensation. Before each electrochemical measurement, the GC disk electrode was polished using SiC P4000 foil.

Each recorded cyclic voltammetry of the reduction and the oxidation process of a compound at a given scan rate is analyzed by measuring i_{p,a} = anodic peak current ; i_{p,c} = cathodic peak current ; v = scan rate ; E_{p,a} = anodic peak potential ; E_{p,c} = cathodic peak potential ; ΔEₚ = E_{p,a}-E_{p,c} ; the apparent standard redox potential E^{0'} = (E_{p,a}-E_{p,c})/2.

Linear sweep voltammetry (LSV) using the RDE at various rotation speed (400 up to 1 000 RPM) were performed to determine the averaged half-wave potential E_{1/2}.

The oxidation (DO) and the reduction (DR) diffusion coefficients were calculated using Randles-Sevcik (RS) equation iₚ = 0.4463.n^{3/2}.F^{3/2}.A.C.v^{1/2}.D^{1/2}.R^{-1/2}.T^{-1/2} = Cte.v^{1/2}.D^{1/2}, n = number of electrons exchanged during the redox process ; F = faradaic number = 96485 C.mol⁻¹ ; A = electrode active surface ; C = redox active molecule concentration ; R = gas constant = 8.314 J.K⁻¹.mol⁻¹ ; T = bulk temperature = 299 K. The experiments are conducted by cyclic voltammetry at various scan rates ranging from 10 mV/s up to 20 V/s.

A graphic of li_{p,a}l and li_{p,c}l = f(Cte.v^{1/2}) is plotted, if the redox process is reversible and diffusion controlled, the peak current should be proportional to the square root of the scan rate. The diffusion coefficients (oxidation and reduction) are then calculated by taking the square of the regression lines slopes. Diffusion coefficients (oxidation and reduction processes) are evaluated by plotting the peak current *versus* a constant multiplied by the square root of the scan rate. The plots regression line gives a straight line with perfect correlation coefficient (100%) confirming that the redox process is reversible and diffusion controlled. The square of each slope gives the associated coefficient diffusion in cm².s⁻¹.

The apparent heterogeneous rate constant during reduction (k^{0'}_{O}) process was calculated using Gileadi's equation (C. Rüssel, W. Jaenicke, Electrochimica Acta, Vol 27, N°12, 1745-1750, 1982) log k^{0'} = -0.48.α + 0.52 + log[(n.F.α.v_{c}.D)/(2.303.R.T)]^{1/2} ; v_{c} is the critical scan rate, it is calculated by plotting the peak potentials *versus* the logarithm of the scan rates, and is more precise when a very wide range of scan rates is used, which is the case here (0.010 up to 20 V.s⁻¹), it is determined at the x-axis of the point of intersection of the regression lines drawn at low and high scan rates; α is the transfer coefficient that is supposed to be close to 0.5 for all presented compounds.

Peak-to-peak separation ΔEₚ values were determined from the third cycle of each cyclic voltammogram and are gathered in the following table 2.

**Table 2: Peak-to-peak separation ΔEₚ of compounds A, B and of one compound of formula (I) at pH 14 (compound B-M3CH) at a concentration of 5.10⁻³ M at a scan rate of 100 mV.s⁻¹**

| Compounds | ΔEₚ (mV) at pH 14 |
|---|---|
| A | 56 |
| B | 40 |
| B-M3CH | 30 |

The standard redox process reversibility evaluation is performed in diluted state, at 5 mM of concentration, at pH 14 buffered with 1 M of KOH. Alan J. Bard et al. (A. J. Bard and L. R. Faulkner, Electrochemical Methods, Wiley, New York, 2nd edition, 2001) presents the 5 criteria defining the reversibility of a redox process in the handbook of electrochemical methods:
i) the peak current absolute values must be proportional to the square root of the scan rate;
ii) the peak potentials must be independent at all scan rates;
iii) the potential peak difference must be equal to 59/n if no protons are involved, n = number of electrons involved in the redox process (2 electrons in this case);
iv) the ratio of peak current absolute values must be equal to the unity;
v) the apparent standard redox potential (E^{0'}) must be equal to the half-wave potential (E_{1/2}).

Main electrochemical properties of compound of formula (I) are gathered in table 3.

**Table 3: Electrochemical parameters of compounds A and B and one compound of formula (I) (compound B-M3CH)**

| Compound (I) | E^{0'} (V *vs* NHE) | E_{1/2} (V *vs* NHE) | D_{O} x 10⁻⁶ (cm²/s) | k^{0'}_{O} x 10^{- 2} (cm/s) |
|---|---|---|---|---|
| A | -0.59 | -0.59 | 3.65 | 0.78 |
| B | -0.57 | -0.57 | 2.99 | 0.80 |
| B-M3CH | -0.49 | -0.49 | 4.58 | 2.47 |

Redox potentials of compound of A and B are are close to -0.6 V *vs* NHE which allows to reach an open-circuit-voltage (ocv) of about 1.1 V at 50% state-of-charge (SOC) in a cell involving standard ferricyanide/ferrocyanide redox couple (E^{0'} = 0.5 V *vs* NHE) in the posolyte.

Redox potentials of compounds of formula (I), i.e. compound B-M3CH, are close to 0.50 V *vs* NHE which allows to reach an ocv of about 1.0 V at 50% SOC in a cell involving standard ferricyanide/ferrocyanide redox couple in the posolyte.

The redox process is fast and reversible for a compound of formula (I). This result is important as it discloses the ability of the redox active material to undergo multiple charge and discharge processes without changes of electrochemical properties and performances.

The diffusion coefficients values vary from 3 to 5 x 10⁻⁶ cm²/s which is typical of redox active materials used in aqueous redox flow battery (ARFB) negolytes in aqueous medium. Inversely, the electron transfer kinetics (_{O} stands for oxidant, ⁰ stands for standard, ^{0'} stands for standard apparent) obtained with compounds of formula (I) (>10⁻² cm/s) are faster than most redox active materials used in ARFB negolytes (see table 4).

**Table 4: Diffusion coefficients and electron transfer kinetics of various redox-active molecules used in negolytes in ARFBs**

| Redox active material in negolytes | D_{O} x 10⁻⁶ (cm²/s) | k^{0'}_{O} x 10⁻² (cm/s) |
|---|---|---|
| DHBQ | 3.66 | 0.21 |
| DHAQ | 4.8 | 0.70 |
| DBEAQ | 1.58 | 0.70 |
| DPPEAQ | 1.37 | |
| Flavine FMN-Na | 1.3 | 0.53 |
| AQ-2,7-DS | 3.8 | 0.72 |
| BTMAP-Vi | 3.3 | 2.2 |
| V³⁺/V²⁺ | 4 | 0.0017 |

### 5. Cycling tests in AORFB environment

The use of a very specific redox flow battery system is essential to allow the energy harvesting of these bio-fermented active molecules. The requisite know-how relates to a set of multiple technological bricks that have been developed together to fit the specifications imposed by organic-based electrolytes in AORFBs.

The electrolyte composition (osmotic flux management, pH buffering, ionic conductivity optimization...); the operational conditions (cycling conditions, in-situ analytical procedures...); the materials choice (pretreatments, electrode material, membrane nature, the interaction with the chemicals and the environment...); the process (hydraulic circuit, impact of the system layout on the performance, the tank design...); the system control (process parameter monitoring, BMS...); the stack design (architecture, materials suitability, electrode design, shunt current management...) represent a non-exhaustive list of specific knowledges that, in synergy, allow the development of a redox flow battery that is compatible with organic-based electrolytes.

In addition, specific know-how in the AORFB field allows to evaluate such electrolyte composed with a bio-fermented organic active material under near-real operational conditions. Namely, the cycling tests are performed during a relevant period (above 10 days of constant cycling which is the average RFB cycling period in the state-of-the-art) ; using very low (relevant with the system's scale) electronic excess in the non-limiting capacity compartment; using very low volume excess (relevant with the system's scale) in the non-limiting capacity compartment ; using low flow rates (relevant with the system's scale) ; reaching more than 4 hours of energy storage in discharge ; using power constant mode during cycling rather than galvanostatic mode.

### Experimental procedure

The electrochemical diagnosis of the compounds A, B, and B-M3CH that was performed in diluted state in a standard three-electrode electrochemical cell was followed by an evaluation in concentrated state (≥0.1 M) where most secondary reactions linked to the redox active degradation are likely to occur.

**General procedure:** Cell tests were carried out on a BCS-815 battery cycler (Biologic) at room temperature. The redox flow battery cell was composed of an Aquivion^{®} membrane selected from E98-09S Solvay (80 microns thickness) or E98-05 Solvay (50 µm thickness) serving as separator sandwiched between two GFD porous graphite felts (SGL Carbon group, GFD 4.6 EA), 4.6 mm thick with an active area of 5 or 25 cm². The electrodes were inserted into 3 mm PVC frames surrounded by EPDM gaskets. The current collectors were made of graphite composite material provided by SGL and the assembly was compressed by two PVC end-plates. The electrolytes were pumped within the cells through remote-control diaphragm liquid dosing pumps (KNF) at a flow rate of 100 mL/min. The tanks were made of polyethylene connected to polyurethane tubing (4 mm inner diameter). Before each test, the cell materials (membrane and electrodes) were carefully rinsed by using 100 mL of distilled water in each tank that circulates into the cell for one hour. The cell, the process and the tanks were air purged and another rinsing step were performed for one hour before air-purging the whole system. The negolyte, which charged (reduced) form is sensitive to oxygen, was protected by an argon blanket atmosphere by bubbling the gas directly into the negolyte that was kept under circulation for 10 minutes.

Capacity utilization was calculated by determining the ratio of the discharge capacity to the theoretical capacity available in the electrolytes. Coulombic efficiency was calculated by determining the ratio of the discharge capacity to the charge capacity. Energy efficiency was calculated by determining the ratio of the discharge energy to the charge energy. The energy density was calculated by determining the ratio of the discharge energy to the total volume of posolyte (positive electrolyte) and negolyte (negative electrolyte).

Under galvanostatic mode, when not specified, the standard operating current density used was 40 mA/cm².

Under power constant mode, when not specified, the standard operating current density used was 40 mW/cm² and a constant voltage step was used at the end of each charge at a specific upper voltage limit.

### Compound A

### ▪ Cycling test

This cell test was performed using materials described above and with the following parameters:
- Active area: 25 cm²
- Membrane: Aquivion^{®} with 80 microns thickness
- Flowrate: 100 mL/min
- Negative pole: 40 mL of negolyte comprising 0.24 M of compound A, 0.9 M of KOH and 0.3 M NaOH
- Positive pole: 42 mL of posolyte comprising 0.6 M potassium ferrocyanide and 0.3 M NaOH
- Averaged pH: 13.5
- Cycling test: galvanostatic mode or power static mode
- Voltage limits: between 0.7 and 1.4 V under galvanostatic mode. Under power static mode, the lower voltage limit was 0.8 V. The upper voltage limit was selected between 1.2 and 1.23 V which, in each case, was followed by a CV step at the same voltage until 0.25 W limit is reached.

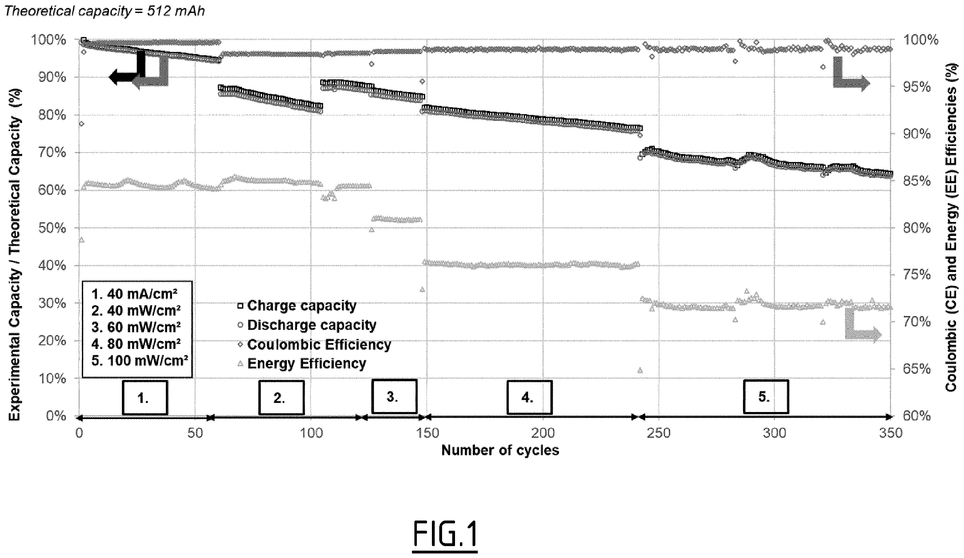

Figure 1 presents the performances of the 350 first cycles. Figure 1 is a graph representing (squares) the charge capacity, (circles) the discharge capacity, (diamonds) the coulombic and (triangles) the energy efficiencies of a cell test involving a negolyte with compound A.

Various cycling conditions were used all along this battery test. First, a galvanostatic mode (40 mA/cm²) was used for 58 cycles. Then, a power static mode was used (40 mW/cm²) with a cutoff upper voltage of 1.2V during 44 cycles. In order to harvest more capacity, a cutoff voltage was then set at 1.23V and various power densities were tested i.e. 40 mW/cm² for 21 cycles; 60 mW/cm² for 22 cycles; 80 mW/cm² for 94 cycles and, finally, 100 mW/cm² for 131 cycles.

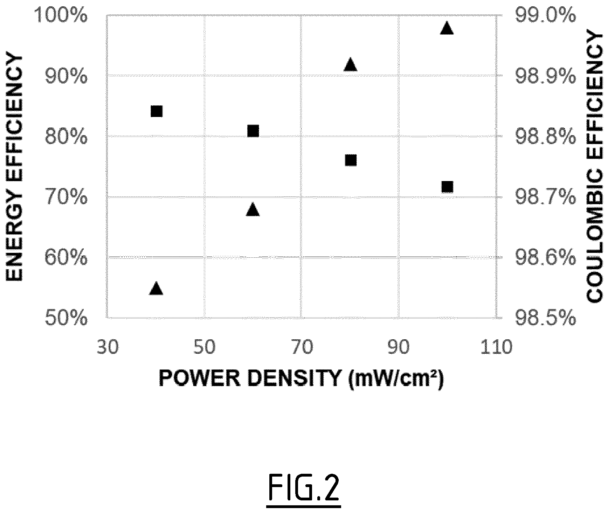

All available capacity (capacity utilization) was reached (100%) at the beginning of the cycling test (which makes sense as the electrochemical kinetics of redox active materials are fast and the important flowrate decrease mass transport limitations) whereas a steady capacity loss (between 0.042% loss and 0.123% capacity loss per cycle) was observed during each cycling step, whatever the current and power densities. In addition, rather low coulombic efficiencies (<99%) were observed during cycles under power static mode while high (>70%) energy efficiencies were observed even at very high nominal power densities (100 mW/cm²), showing that very high nominal power densities can be used with such systems, see figure 2.

Figure 2 represents the evolution of (dark triangles) coulombic and (dark squares) energy efficiencies according to the nominal power density during the same cycling test involving a negolyte containing compound A.

These 2 observations (important capacity loss/cycle and low coulombic efficiencies) indicate that there is likely an unwanted secondary reaction that occurred during the test (we excluded osmosis, cross-over, electrolysis or air tightness failure problematics).

Furthermore, at 50% SOC, where the system does not suffer from charge-transfer or mass-transport limitations, a linar polarization curve (LPC) was regularly performed during the cycling test in order to track the active surface resistance (ASR) of the cell. The LPC was always realized in charge and discharge using a ramp of 100 A/min and -100 A/min from 0 to 2 A and from 0 to -2 A in order to evaluate the active surface resistance of the cell.

The active surface resistance was quite stable but fluctuated around 2.0-2.3 Ω.cm², which was consistent with an electrochemical cell comprising a Nafion-type membrane with a thickness of 80 µm which diffuses a majority of potassium cations (alkaline medium).

### ▪ Post-cycling analyses

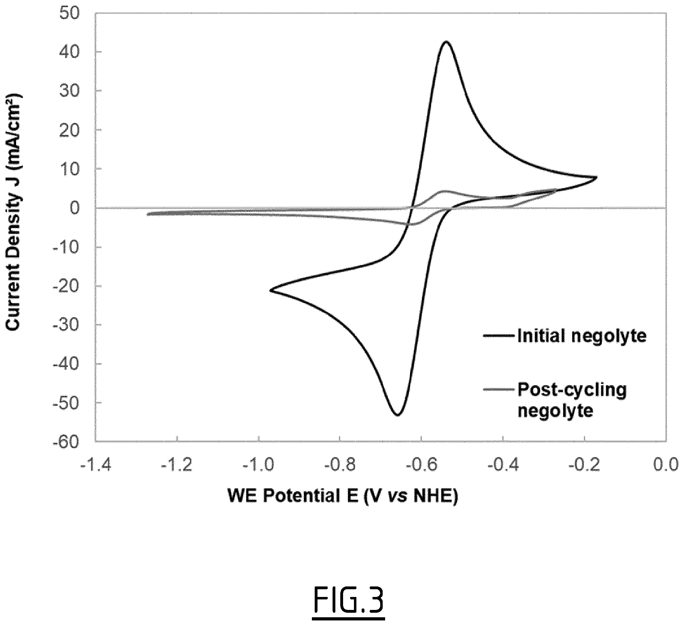

A preliminary electrochemical test was performed on the negolyte before and after the cycling test (figure 3).

The typical electrochemical signature of compound A was registered before the cycling test. The redox signal was centered at -0.83 V *vs* Ag/AgCl (or -0.60 V *vs* NHE) with a max current of roughly -4 mA during reduction step and a max current of roughly 3 mA during reoxidation step. After cycling, the redox signature corresponding to the compound A is decreased in current intensity with a factor x10. The osmosis only can't explain this major signature decrease. Also, an oxidation occurs at -0.57 V *vs* Ag/AgCl that was not originally present in the negolyte.

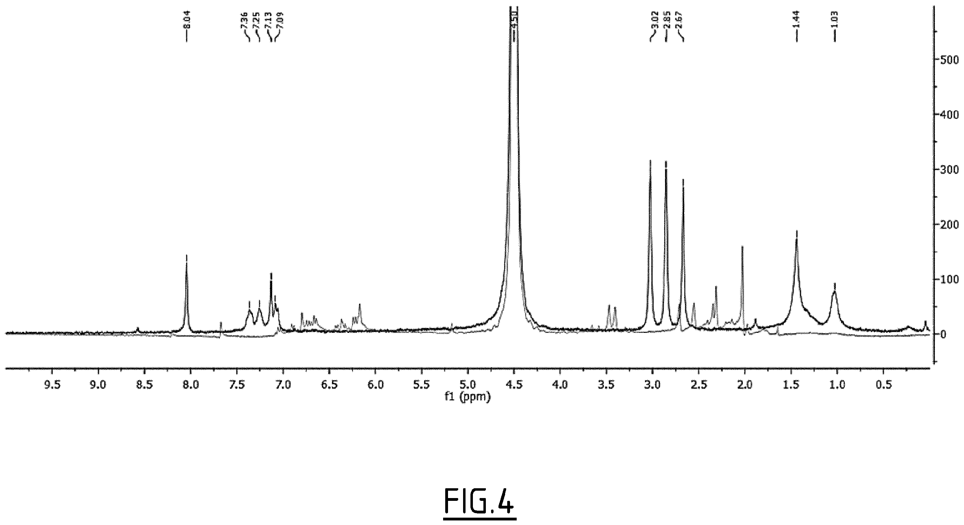

Further analyses were performed using ¹H NMR spectroscopy, see figure 4.

The protons signature is very different. The aromatic protons situated between 7.4 and 7.7 ppm, representative of the anthraquinone structure, are partly present after cycling but additional peaks appear between 6.4 and 7.4 ppm. The protons attached to the methyl group presents at 3.31 ppm before cycling, disappears after cycling while 2 singlets appear at 3.70 and 3.76 ppm. These very different protons signature suggest that the compound A was degraded into multiple compounds during the cycling test.

Considering these analyses, the compound A allows to reach high nominal power densities (100 mW/cm²) with decent energy densities (3.7 Wh/L) but a low chemical stability was highlighted by multiple indicators and implies that this compound is not adapted to the application in such ionic environment using such cycling conditions.

### Compound B

### ▪ Cycling test

This cell test was performed using materials described above and with the following parameters:
- Active area: 5 cm²
- Membrane: Aquivion^{®} with 80 microns thickness
- Flowrate: 100 mL/min
- Negative pole: 20 mL of negolyte comprising 0.18 M of compound B and 1.4 M of KOH
- Positive pole: 50 mL of posolyte comprising 0.2 M potassium ferrocyanide and 1 M KOH
- Averaged pH: 14.0
- Cycling test: galvanostatic mode
- Voltage limits: The upper cutoff voltage was fixed at 1.31 V (current density fixed at 40 mA/cm²) while the lower cutoff voltage depended on the nominal current density i.e. 0.75 V or 0.80 V when running at 40-80 mA/cm² and 0.70 V when increasing the current density up to 120 mA/cm².

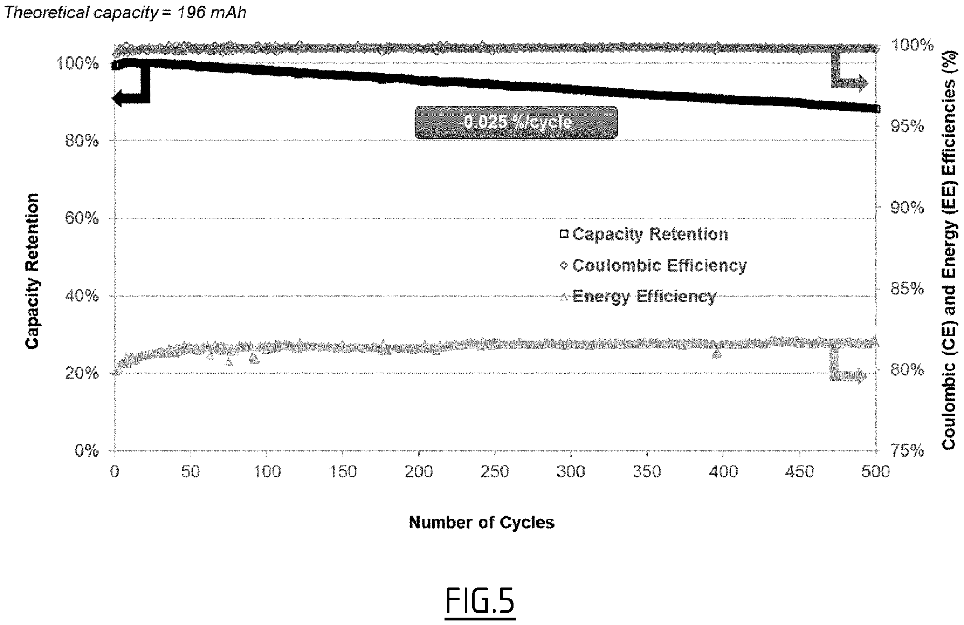

Figure 5 presents 500 typical cycles of this battery test.

Figure 5 is a graph representing (squares) the capacity retention, (diamonds) coulombic and (triangles) energy efficiencies of a cell test involving a negolyte with compound B. 500 cycles are represented, the current densities in discharge and charge were fixed at 40 mA/cm² with 0.80 to 1.40 V cutoff voltages.

The experimental capacity achieved was 78% at the beginning of the cycling test whereas a steady capacity loss of 0.025% per cycle was observed. High average coulombic efficiencies were recorded during these 500 cycles (∼99.82%) with high (∼81.47%) average energy efficiencies.

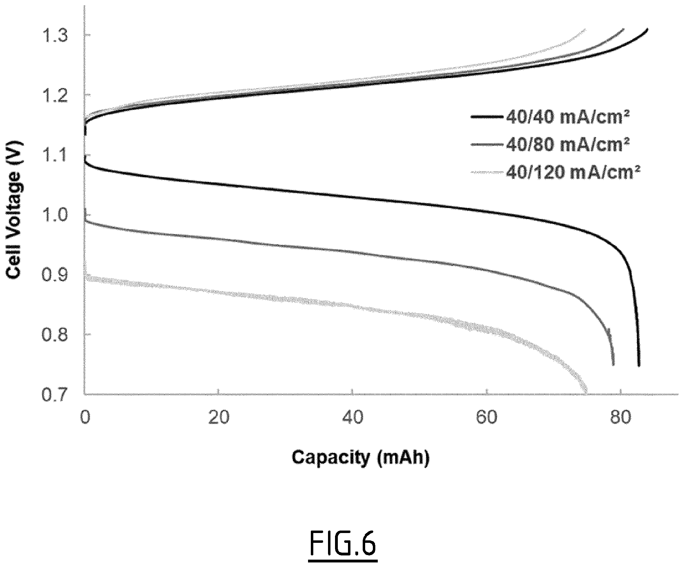

The battery was then further operated and different current densities were tested, see figure 6.

Figure 6 is a graph representing the capacity available during charge at 40 mA/cm² and during discharge (dark) at 40, (dark grey) at 80 and (light grey) at 120 mA/cm² of a cell test involving a negolyte with compound B

Air sealing problematics were encountered during this battery test (due to very low electrolyte volumes in small tanks that are difficult to perfectly seal), which lowered the available capacity (we excluded cross-over, electrolysis or irreversible chemical degradation problematics) below 40%. Still, despite this problematic, we can observe that such battery can be cycled using high nominal current densities.

Furthermore, at 50% SOC, the ASR was tracked during the cycling test using standard procedure, The active surface resistance was quite stable but fluctuated around 2.0-2.2 Ω.cm², which was consistent with an electrochemical cell comprising a Nafion-type membrane with a thickness of 80 µm which diffuses a majority of potassium cations (alkaline medium).

### ▪ Post-cycling analyses

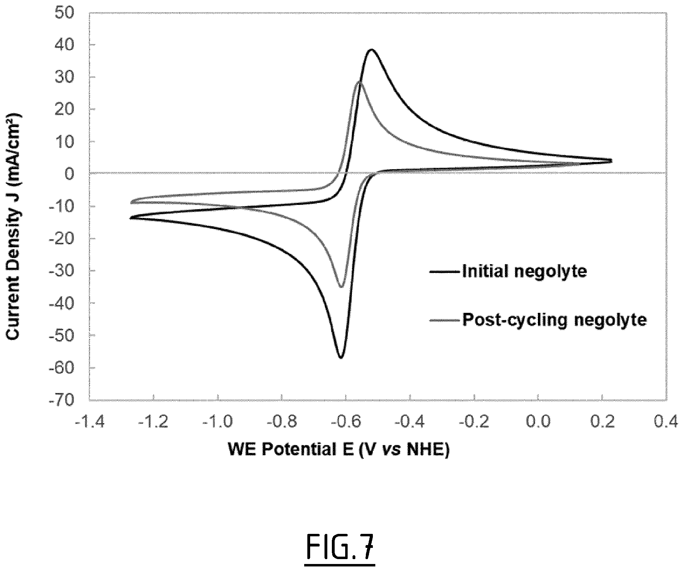

A preliminary electrochemical test was performed on the negolyte before and after thousands of cycles and a few months of cycling test (figure 7).

The typical electrochemical signature of compound B was registered before the cycling test. The redox signal was centered at -0.80 V *vs* Ag/AgCl (or -0.57 V *vs* NHE) with a max current of roughly -4 mA during reduction step and a max current of roughly 2.8 mA during reoxidation step. After cycling, the redox signature corresponding to the compound B is a bit decreased in current intensity, about 30-35%, which corresponds to the water transfer (osmosis) from the posolyte to the negolyte during the cycling test that has diluted the negolyte and thus, its redox signature intensity.

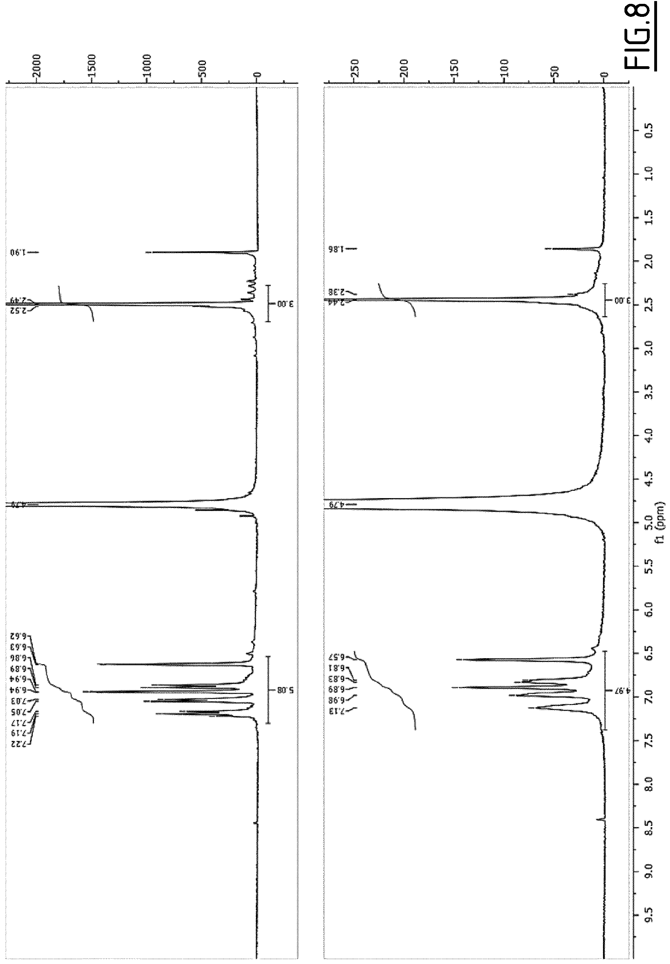

Further analyses were performed using ¹H NMR spectroscopy, see figure 8.

The protons signatures are similar. The aromatic protons are situated between 6.6 and 7.2 ppm. The protons attached to the methyl group are located at 2.5 ppm. No additional peaks are observed. This result suggests that the compound B does not undergo any degradation during the cycling test.

Considering these analyses, the compound B allows to reach high nominal current densities (120 mA/cm²) with rather low energy densities (2.1 Wh/L). A small and steady capacity loss of -0.025% per cycle is recorded but is associated to air tightness failure (low electrolyte volumes and small tanks) and/or reversible disproportionation reaction (see 2,6-dihydroanthraquinone behavior in alkaline environment, M. J. Aziz et al., J. Am. Chem. Soc., 2019, 141, 20, 8014-8019).

### Compound B-M3CH

### ▪ Cycling test

This cell test was performed using materials described above and with the following parameters:
- Active area: 25 cm²
- Membrane: Aquivion^{®} with 50 microns thickness
- Flowrate: 100 mL/min
- Negative pole: 40 mL of negolyte comprising 0.1 M of compound B-M3CH, 0.225M KOH,0.042 M NaOH, 0.3M KCI and 0.05M of NaCl
- Positive pole: 44 mL of posolyte comprising 0.15 M potassium ferrocyanide, 0.05 M sodium ferrocyanide, 0.075M KOH and 0.025 M of NaOH
- Averaged pH: 13.0
- Cycling test: power static mode
- Voltage limits: under power static mode, the lower voltage limit was 0.7 V. The upper voltage limit was fixed at 1.25 V which and was followed by a CV step at the same voltage until 0.25 W limit is reached.

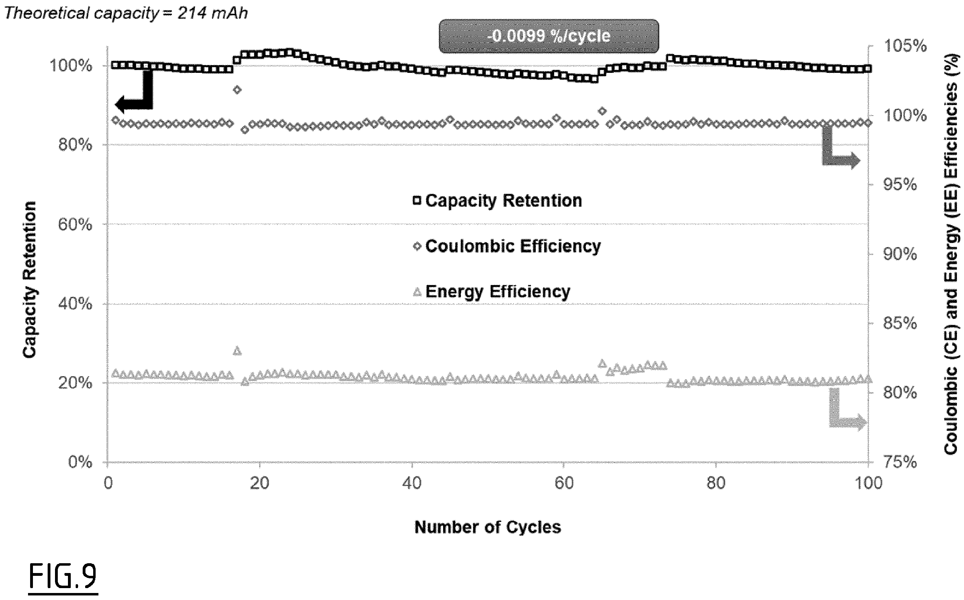

Figure 9 presents 100 typical cycles of this battery test among more than 1000 cycles.

Figure 9 is a graph representing (squares) the capacity retention, (diamonds) coulombic and (triangles) energy efficiencies of a cell test involving a negolyte with compound B-M3CH. 100 cycles are represented, the power densities in discharge and charge were fixed at 40 mW/cm².

The experimental capacity achieved was 70% at the beginning of the cycling test whereas a steady capacity loss of 0.0099% per cycle was observed. High average coulombic efficiencies were recorded during these 500 cycles (∼99.41%) with high (∼81.17%) average energy efficiencies.

Air sealing problematics were encountered during this battery test (due to low electrolyte volumes in small tanks that are difficult to perfectly seal), which is partly responsible for the observed capacity loss (we excluded cross-over, electrolysis or irreversible chemical degradation problematics).

Furthermore, at 50% SOC, the ASR was tracked during the cycling test using standard procedure. The ASR was quite stable, at 2.0 Ω.cm², which was consistent with an electrochemical cell comprising a Nafion-type membrane with a thickness of 50 µm which diffuses a mixture of potassium and sodium cations (alkaline medium).

### ▪ Post-cycling analyses

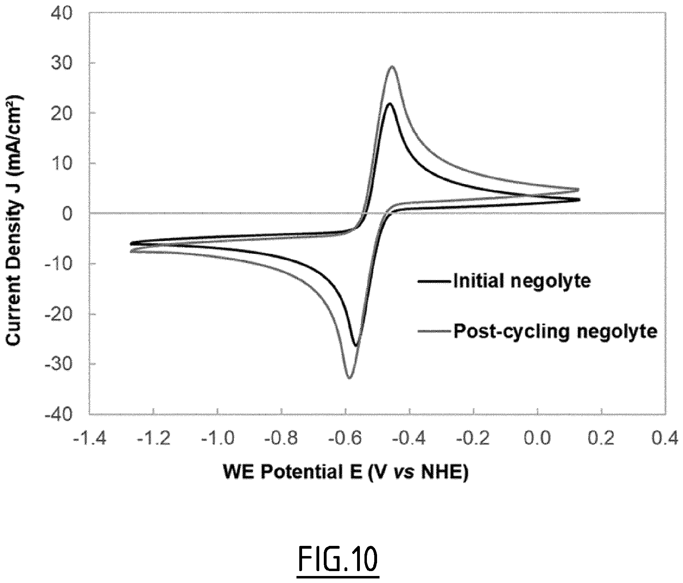

A preliminary electrochemical test was performed on the negolyte before and after the cycling test (figure 10) which lasted for more than 1000 cycles, or 25 days of constant cycling.

The typical electrochemical signature of compound B-M3CH was registered before the cycling test. The redox signal was centered at -0.75 V *vs* Ag/AgCl (or -0.52 V *vs* NHE) with a max current of roughly -2.3 mA during reduction step and a max current of roughly 2.0 mA during reoxidation step. After cycling, the redox signature corresponding to the compound B-M3CH has increased in current intensity, about 30%, which corresponds to the water transfer (osmosis) from the negolyte to the posolyte during the cycling test that has concentrated the negolyte and thus, increased the redox signature intensity of B-M3CH compound.

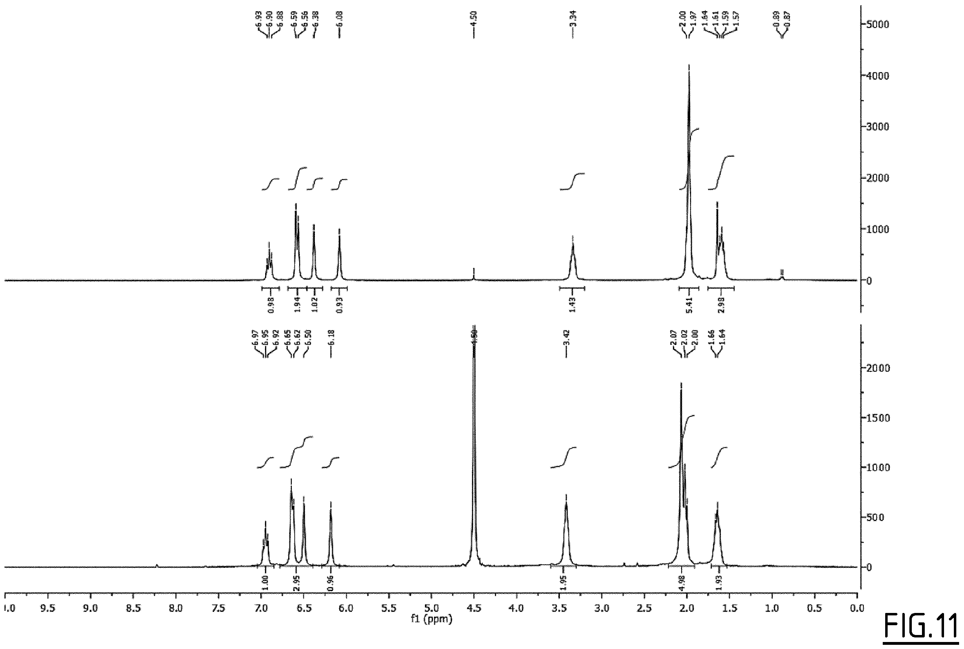

Further analyses were performed using ¹H NMR spectroscopy, see figure 11.

The protons signatures are similar. The aromatic protons are situated between 6.2 and 7.3 ppm. The protons attached to the (CH2)3 alkyl chain are located at 1.8-1.9, 2.2-2.3 and at 3.5-3.7 ppm. No additional peaks are observed. This result suggests that the compound B-M3CH does not undergo any degradation during the cycling test.

Considering these analyses, the compound B-M3CH allows to reach good nominal current densities (40 mA/cm²) with rather low energy densities (2.0 Wh/L). A steady capacity loss of -0.0099% per cycle is recorded but is associated to air tightness failure (low electrolyte volumes and small tanks). This result shows that the chemical modification of compound B into compound B-M3CH has increased its stability in the application. This effect can be explained by the increase of redox potential of the anthraquinone though the alkylation of one phenolic group that may stabilize of its reduced state (see 2,6-dihydroanthraquinone behavior in alkaline environment, M. J. Aziz et al., J. Am. Chem. Soc., 2019, 141, 20, 8014-8019).

## Claims

1. A redox flow battery comprising at least one aqueous electrolyte comprising a fermented compound of formula (I) 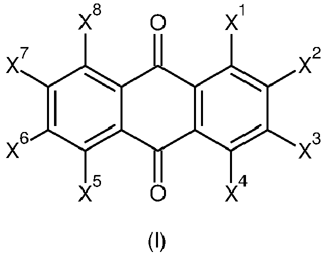 and/or an ion of compound (I),
and/or a salt of compound (I),
and/or a reduced form of the anthraquinone member of compound (I),
wherein:
X₁, X₂, X₃, X₄, X₅, X₆, X₇ and X₈ are independently selected from the group consisting of a hydrogen atom, a halogen atom, an ether group of formula -O-A, a linear, cyclic or branched, saturated or unsaturated, optionally substituted, hydrocarbon group comprising from 1 to 10 carbon atoms, a OH group, a -R₁ group and a -O-A-R₁ group,
A representing a linear, cyclic or branched, saturated or unsaturated, optionally substituted, hydrocarbon group comprising from 1 to 10 carbon atoms;
R₁ representing COOH or SO₃H;
wherein one to three of X₁, X₂, X₃, X₄, X₅, X₆, X₇ and X₈ is OH, and
wherein one and only one of X₁, X₂, X₃, X₄, X₅, X₆, X₇ and X₈ is -O-A-R₁.

2. The redox flow battery according to claim 1, wherein one and only one of X₁, X₂, X₃, X₄, X₅, X₆, X₇ and X₈ is OH and wherein one and only one of X₁, X₂, X₃, X₄, X₅, X₆, X₇ and X₈ is -O-A-R₁.

3. The redox flow battery according to claim 1 or 2, wherein one and only one of X₃ and X₈ is OH, and one and only one of X₃ and X₈ is -O-A-R₁.

4. The redox flow battery according to claim 1 or 2, wherein X₃ is OH and X₈ is -O-A-R₁.

5. The redox flow battery according to claim 1 or 2, wherein X₃ is -O-A-R₁ and X₈ is OH.

6. The redox flow battery according to claim 1, wherein two of X₃, X₆, and X₈ are OH, and wherein one and only one of X₃, X₆ and X₈ is -O-A-R₁.

7. The redox flow battery according to claim 1, wherein three of X₃, X₅, X₆, and X₈ are OH, and wherein one and only one of X₃, X₅, X₆ and X₈ is -O-A-R₁.

8. The redox flow battery according to any one of claims 1 to 7, wherein X₁ represents a linear, cyclic or branched, saturated or unsaturated, optionally substituted, hydrocarbon group comprising from 1 to 10 carbon atoms, preferably from 1 to 3 carbon atoms, and preferably represents a methyl group.

9. The redox flow battery according to any one of claims 1 to 8, wherein A represents (CH₂)ₙ, optionally substituted, wherein n is an integer selected from 1 to 10, preferably selected from 2 to 5 and more preferably selected from 3 to 4.

10. The redox flow battery according to any one of claims 1 to 8, wherein said - O-A-R₁ group represents O-(CH₂)ₙ-COOH or O-(CH₂)ₙ- SO₃H wherein n is from 1 to 10.

11. The aqueous electrolyte according to any one of claims 1 to 8, wherein R₁ is selected from the group consisting of CO₂H, CO₂⁻M⁺, SO₃H and SO₃⁻M⁺, M⁺ being selected from the group consisting of Li⁺, Na⁺, K⁺ and NH₄⁺, preferably M⁺ is Na⁺ or K⁺.

12. The redox flow battery according to any one of claims 1 to 11, wherein X₂ represents a -R₁ group, and preferably represents COOH or a salt thereof.

13. The redox flow battery according to any one of claims 1 to 12, wherein said compound of formula (I) has the following structure: 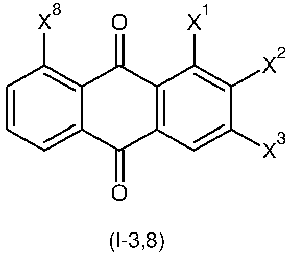 wherein X₁, X₂, X₃, and X₈ are as defined in any one of claims 1 to 12.

14. The redox flow battery according to any one of claims 1 to 13, wherein said compound of formula (I) has the following structure: 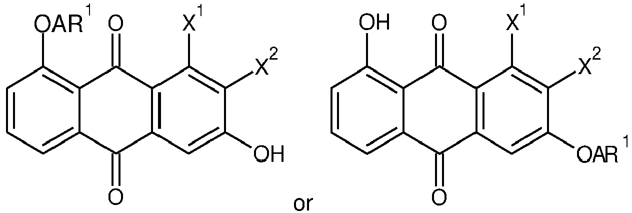 wherein X₁, X₂ and O-A-R₁ are as defined in any one of claims 1 to 12.

15. The redox flow battery according to any one of claims 1 to 14, wherein said compound of formula (I) has the following structure: 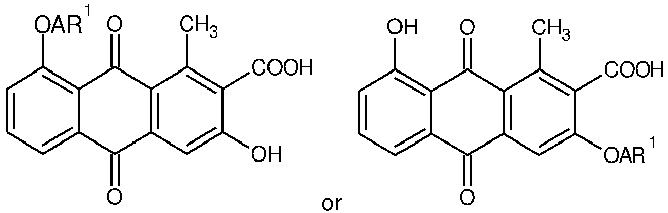 wherein O-A-R₁ are as defined in any one of claims 1 to 12.

16. The redox flow battery according to any one of claims 1 to 14, wherein said compound of formula (I) has the following structure: 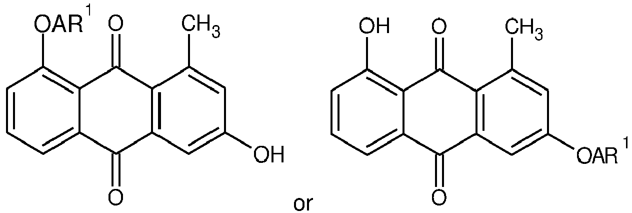 wherein O-A-R₁ is as defined in any one of claims 1 to 12.

17. Use of an aqueous electrolyte according to any one of claims 1 to 16, as a negative electrolyte in a redox flow battery.

18. A method for generating electricity by at least one redox flow battery, wherein the redox flow battery is defined according to any one of claims 1 to 16.
